# EUROPÄISCHE PATENTSCHRIFT

(11) **EP 2 688 458 B1**
(45) Veröffentlichungstag und Bekanntmachung des Hinweises auf die Patenterteilung: **27.07.2022**
(21) Anmeldenummer: 12700151.9
(22) Anmeldetag: 03.01.2012
(51) Int. Cl.: A61B 3/00, A61B 18/20, A61F 9/008

(54) **OPHTHALMOLOGISCHES GERAET**
OPHTHALMOLOGICAL DEVICE
APPAREIL OPHTALMOLOGIQUE

(30) Priorität: 25.03.2011 DE 102011006085; 25.03.2011 US 201161467712 P
(43) Veröffentlichungstag der Anmeldung: 29.01.2014
(73) Patentinhaber: Carl Zeiss Meditec AG, 07745 Jena (DE)
(72) Erfinder: SONDERMANN, Mario, 01731 Quohren (DE); HANFT, Marco, 07743 Jena (DE); DOERING, Dirk, 99094 Erfurt (DE)
(74) Vertreter: Gleim Petri Oehmke Patent- und Rechtsanwaltspartnerschaft mbB
(86) Internationale Anmeldenummer: PCT/EP2012/050035
(87) Internationale Veröffentlichungsnummer: WO 2012/130480

(56) Entgegenhaltungen:
- WO-A1-2008/087483
- WO-A1-2010/142311
- US-A1- 2009 299 347

## Beschreibung

### Gebiet der Erfindung

Die vorliegende Erfindung bezieht sich auf ein ophthalmologisches Gerät zur Anwendung von Laserstrahlung am und im Auge, insbesondere zur Untersuchung und/oder laserchirurgischen Behandlung der Augenhornhaut und der Augenlinse.

### Stand der Technik

Ophthalmologische Geräte dienen beispielsweise dazu, die Fehlsichtigkeit des menschlichen Auges durch einen laserchirurgischen Eingriff an der Augenhornhaut zu korrigieren. Von besonderer Bedeutung ist dabei das unter der Bezeichnung "LASIK" (Laser in situ Keratomileusis) bekannte Verfahren, bei dem mittels eines gepulsten Laserstrahls Material nicht an der Oberfläche der Augenhornhaut, sondern im Inneren der Augenhornhaut abgetragen wird. Dazu wird an der äußeren Oberfläche der Augenhornhaut ein auch als "Flap" bezeichneter, klappenartiger Deckel gebildet, dessen Dicke wesentlich geringer ist als die Dicke der Augenhornhaut. Zur eigentlichen Ablationsbehandlung wird dieser Deckel weggeklappt, woraufhin von der Oberfläche des nun freigelegten Bereichs mittels eines gepulsten Laserstrahls Material zur Korrektur der Fehlsichtigkeit abgetragen wird. Danach wird der Deckel auf die ablatierte Oberfläche zurückgeklappt.

Um den Deckel in genau definierter Tiefe in der Augenhornhaut möglichst schonend und präzise herstellen zu können, ist vorgeschlagen worden, Femtosekundenlaserpulse, d.h. Laserpulse mit Pulsbreiten kleiner als 10⁻¹²s, zu verwenden. Mittels solcher Pulse können in der Augenhornhaut optische Durchbrüche, auch Photodisruptionen genannt, erzeugt werden, die lokal begrenzt sind und eine Ausdehnung von nur wenigen Mikrometern aufweisen. Durch eine dichte Positionierung einer Vielzahl solcher optischer Durchbrüche an genau vorgegebenen Stellen kann der Deckel sehr exakt ausgebildet werden kann. Eine wesentliche Voraussetzung für die genaue Ausbildung des Deckels ist dabei die exakte Positionierung des Fokus des gepulsten Laserstrahls nicht nur in lateraler Richtung, sondern vor allem auch in der Tiefe der Augenhornhaut, also in Ausbreitungsrichtung des Laserstrahls. Diesbezüglich beschreibt DE 10 2005 013 949 A1 eine Scanvorrichtung zur Fokussierung eines Strahlenbündels in ein vorgegebenes Volumen sowie ein Verfahren zur Fokussierung eines Strahlenbündels in ein bestimmtes Volumen. Diese Scanvorrichtung ist insbesondere zur Verwendung in Geräten geeignet, die zur laserchirurgischen Behandlung der Augenhornhaut mit im Femtosekundenbereich gepulster Laserstrahlung vorgesehen sind. Sie ermöglicht es, das Laserlicht in beliebige Antastpunkte im Bereich der Augenhornhaut zu fokussieren.

In DE 10 2008 027 358 A1 ist ein Lasersystem beschrieben, das in ophthalmologischen Geräten zur Analyse und Behandlung der Augenlinse verwendet werden kann. Es hat gegenüber Lasersystemen in vergleichbaren Geräten den Vorteil, dass die Detektion des in der Augenlinse rückgestreuten Laserlichtes mit größerer Genauigkeit möglich ist, so dass eine refraktiv-chirurgische Therapie der Augenlinse mit höherer Präzision vorgenommen werden kann. Zur Therapie wird eine im Femtosekundenbereich gepulste Laserstrahlung in ausgewählte Antastpunkte im Bereich der Augenlinse fokussiert.

In der Druckschrift US 2009/299347 ist ein ophthalmologisches Gerät mit den Merkmalen des Oberbegriffs des Anspruchs 1 gezeigt. Bei diesem Gerät kann die Fokusposition im Bereich der Krümmung der Augenhornhaut in der Tiefe variiert werden.

Der vorbeschriebene Stand der Technik in Bezug auf die medizinische Untersuchung und laserchirurgische Behandlung der an der Peripherie des Auges liegenden Augenhornhaut einerseits und der innerhalb des Auges liegenden Augenlinse andererseits hat den Nachteil, dass lediglich ophthalmologische Geräte zur Verfügung stehen, die lediglich den Anforderungen an ihren jeweiligen speziellen Verwendungszweck genügen. D.h. sie unterscheiden sich bezüglich ihrer Abbildungseigenschaften, insbesondere hinsichtlich Apertur und Fokusgröße, Fokuslage im Auge, Korrektion des Öffnungsfehlers oder Korrektion von Feldfehlern so voneinander, dass sie entweder nur zur Untersuchung und Behandlung der Augenhornhaut oder nur zur Untersuchung und Behandlung der Augenlinse ausgebildet sind.

Das bedeutet gerätetechnischen Aufwand, der einmal aus Gründen der Anschaffungskosten nicht effektiv ist, zum anderen auch deshalb nicht, weil mehrere Einzweckgeräte meist auch nicht ausgelastet sind. Hinzu kommt der zeitliche Aufwand der erforderlich ist, um die verschiedenen Geräten zumindest zur Untersuchung desselben Auges eines Patienten jeweils neu einzurichten.

### Beschreibung der Erfindung

Davon ausgehend liegt der Erfindung die Aufgabe zugrunde ein ophthalmologisches Gerät zu schaffen, das sowohl zur Untersuchung und laserchirurgischen Behandlung der Augenhornhaut als auch der Augenlinse nutzbar ist.

Erfindungsgemäß wird diese Aufgabe gelöst mit einem ophthalmologischen Gerät wie es im unabhängigen Anspruch 1 definiert ist.

Die Variation der Abstände erfolgt durch Verschieben der betreffenden Linsengruppen des Objektivs entlang ihrer optischen Achse. Mit der Variation der Abstände wird neben der Fokusposition auch Einfluss auf die Korrektion der Abbildungsfehler genommen. So ändert sich mit einer Variation des Fokus in der Tiefe die sphärische Aberration, wenn man nicht durch die Linsenbewegungen Ausgleich schafft.

Diese Linsengruppen des Objektivs sind vorteilhaft zwischen zwei Endlagen verschiebbar, wobei diese Endlagen zwei verschiedene, fest vorgegebene Abstände in Richtung der optischen Achse definieren. Eine dieser Endlagen ist der Positionierung des Fokus im Bereich der Augenhornhaut, die andere der Positionierung des Fokus im Bereich der Augenlinse zugeordnet. Im Sinne der Erfindung ist unter Positionierung des Fokus "im Bereich der Augenhornhaut" zu verstehen, dass der Fokus auf beliebige Antastpunkte auf oder in der Augenhornhaut ausrichtbar ist, unter Positionierung des Fokus "im Bereich der Augenlinse" ist zu verstehen, dass der Fokus auf beliebige Antastpunkte auf oder in der Augenlinse ausrichtbar ist. Zwecks Verschiebung von einer in die andere Endlage sind die beweglichen Linsengruppen vorteilhaft mit elektromechanischen Antrieben gekoppelt, die mit einer Steuereinheit in Verbindung stehen.

Die Verschiebung von Linsengruppen des Objektivs entspricht prinzipiell der Umschaltung zwischen zwei Betriebsmodi, wobei in dem einen Betriebsmodus das Gerät zur Untersuchung und Behandlung der Augenlinse, in dem anderen Betriebsmodus zur Untersuchung und Behandlung der Augenhornhaut verfügbar und die der Erfindung zugrunde liegende Aufgabe grundsätzlich gelöst ist.

In einer bevorzugten Ausführungsform ist dem Objektiv ein in Strahlungsrichtung verschiebbares optisches Element mit negativer Brechkraft vorgeordnet, mit dessen Verschiebung die Fokusposition in der Koordinatenrichtung Z variierbar ist, jedoch jeweils nur innerhalb des Bereiches der Augenhornhaut oder innerhalb des Bereiches der Augenlinse. Mit der Verschiebung dieses Elementes ist die Divergenz des Strahlenbündels veränderbar. In Abhängigkeit von dem jeweils eingestellten Divergenzwinkel fokussiert das Objektiv das Strahlenbündel in verschiedene Positionen in Z-Richtung innerhalb des Bereiches der Augenhornhaut oder innerhalb des Bereiches der Augenlinse.

Das verschiebbare optische Element kann beispielsweise als Linse, reflektives Element oder diffraktiv-optisches Element ausgeführt sein. Die Ausbildung erfolgt vorzugsweise als Linse. Um eine kontrollierte Verschiebung entlang der optischen Achse zu ermöglichen, ist die Linse mit einer parallel zum Strahlengang ausgerichteten Geradführung zwangsgekoppelt.

Des Weiteren ist dem Objektiv eine Einrichtung zur Ablenkung der Laserstrahlung in den Koordinatenrichtungen X, Y vorgeordnet, wobei diese laterale Ablenkung ebenfalls jeweils nur innerhalb des Bereiches der Augenhornhaut oder innerhalb des Bereiches der Augenlinse möglich ist.

Die Ablenkeinrichtung kann grundsätzlich in an sich bekannter Art und Weise aufgebaut sein. Beispielsweise kann sie nur einen Spiegel aufweisen, der um zwei voneinander verschiedene, vorzugsweise orthogonale Achsen kippbar ist. Vorzugsweise sind jedoch zwei voneinander beabstandete, relativ zueinander schwingende Spiegel vorgesehen. Ein solcher Aufbau erlaubt in vorteilhafter Weise mit einfachen Mitteln eine sehr genaue Lagerung und Einstellung der Spiegel und damit der lateralen Fokuslage.

Zwischen den beiden Spiegeln kann eine Pupillenoptik angeordnet sein, die den ersten auf den zweiten Spiegel abbildet, wodurch sich eine eindeutige Pupillenlage ergibt. Eine eindeutige und feste Lage der Pupille ist bei präzise arbeitenden optischen Systemen die Voraussetzung für eine gute Korrektion.

Zwischen der Ablenkeinrichtung und dem Objektiv kann ein Strahlteiler angeordnet sein, durch den ein Teil des von der Augenhornhaut oder Augenlinse kommenden Beobachtungslichtes zu Beobachtungs- bzw. Abbildungszwecken ausgekoppelt wird.

Während der Untersuchung oder Behandlung des Auges ist vorteilhaft ein Kontaktglas mit einer konkaven Kontaktfläche auf die Augenhornhaut aufgesetzt, um eine Bewegung des Auges zu unterdrücken. Darüber hinaus wird durch das Kontaktglas die Brechkraft des Auges reduziert und die Korrektion der Foki vereinfacht.

In einer bevorzugten Ausgestaltung der Erfindung ist der verschiebbaren Linse negativer Brechkraft eine Sammellinse nachgeordnet, so dass beide Elemente gemeinsam eine Expanderbaugruppe bilden, die den Durchmesser eines parallelen, in die Linse einfallenden Strahlenbündels um ein vorgegebenes Maß aufweitet. Tritt also ein paralleles Strahlenbündel mit einem Durchmesser d1 in den Expander ein, tritt ein paralleles Strahlenbündel mit einem Durchmesser d2 > d1 aus.

Der Austrittsdurchmesser hat Einfluss auf den augenseitigen Öffnungswinkel. Davon ausgehend liegt es im Rahmen der Erfindung, Mittel zur Veränderung des augenseitigen Öffnungswinkels vorzusehen, beispielsweise indem mehrere Negativlinsen mit unterschiedlichem Divergenzwinkel verfügbar und gegeneinander austauschbar sind.

Der Austausch der Negativlinsen gegeneinander zwecks Erzielung verschiedener augenseitiger Öffnungswinkel kann im Zusammenhang mit dem Versetzen der Fokusposition aus dem Bereich der Augenhornhaut in den Bereich der Augenlinse und umgekehrt vorgenommen werden, oder auch in Abhängigkeit von der Größe des zu untersuchenden bzw. zu behandelnden Auges, wobei die Augengröße vorzugsweise durch die Vorderkammertiefe definiert ist. Der Austausch derartiger Baugruppen kann automatisch oder manuell erfolgen.

Mit dem erfindungsgemäßen Gerät kann vorteilhaft eine schnelle und einfache Einstellung der Fokuslage in drei Dimensionen innerhalb des jeweils vorgegebenen Bereiches - dem Bereich der Augenhornhaut oder dem Bereich der Augenlinse - erzielt werden. Ein Gerätewechsel wie im bisherigen Stand der Technik ist nicht mehr erforderlich.

### Kurze Beschreibung der Zeichnungen

Die Erfindung wird nachfolgend anhand von Ausführungsbeispielen näher erläutert. In den zugehörigen Zeichnungen zeigen:
- Fig.1: eine schematische Darstellung eines ophthalmologischen Gerätes zur Anwendung von Laserstrahlung nach Stand der Technik,
- Fig.2: eine Variante der Anordnung nach Fig.1 mit einem Strahlteiler zur Ablenkung des Beobachtungsstrahlenbündels in Richtung auf einen Tubus mit Okular oder eine Kamera,
- Fig.3: eine schematische Darstellung des erfindungsgemäßen Gerätes zur laserchirurgischen Untersuchung und Behandlung des Auges in einer ersten bevorzugten Ausführungsform,
- Fig.4: eine Variante der Ausführungsform nach Fig.3 mit einem Strahlteiler zur Ablenkung des Beobachtungsstrahlenbündels in Richtung auf einen Tubus mit Okular oder eine Kamera,
- Fig.5: eine schematische Darstellung des erfindungsgemäßen Gerätes zur laserchirurgischen Untersuchung und Behandlung des Auges in einer zweiten bevorzugten Ausführungsform.

### Ausführliche Beschreibung der Zeichnungen

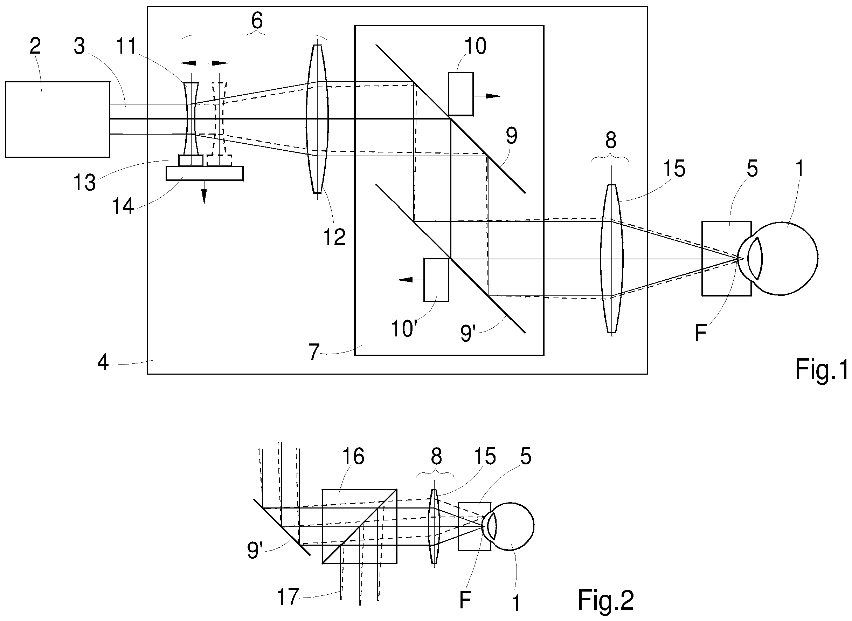

Das in Fig.1 gezeigte, dem Stand der Technik entsprechende Gerät zur Behandlung eines menschlichen Auges 1 umfasst eine Strahlungsquelle 2, die ein Strahlenbündel 3 aus im Femtosekundenbereich gepulster Laserstrahlung abgibt, und eine Scanvorrichtung 4, mit der das Strahlenbündel 3 in ausgewählte Positionen innerhalb des Bereiches der Augenhornhaut fokussiert werden kann. Auf der Augenhornhaut befindet sich ein Kontaktglas 5 mit einer konkaven Kontaktfläche, das Bewegungen des Auges 1 während einer Untersuchung oder Behandlung unterdrückt.

Die Strahlungsquelle 2 ist beispielhaft zur Abgabe von Laserstrahlung im Wellenlängenbereich um 1040 nm mit einer Pulsbreite im Bereich von etwa 200 fs ausgebildet.

Die Scanvorrichtung 4 weist in Richtung des von der Strahlungsquelle 2 ausgehenden Strahlenbündels 3 eine Eingangsoptik 6 auf, welcher in Strahlungsrichtung eine Ablenkeinrichtung 7 folgt. Die Ablenkeinrichtung 7 lenkt das aus der Eingangsoptik 6 austretende Strahlenbündel 3 entsprechend vorgegebener Steuersignale in lateraler Richtung, das heißt in X- und Y-Richtung quer zur Z-Richtung des einfallenden Strahlenbündels 3 ab. Der Ablenkeinrichtung 7 folgt im Strahlengang ein Objektiv 8, welches das Strahlenbündel 3 in den Bereich der Augenhornhaut fokussiert.

Die Ablenkeinrichtung 7 verfügt über zwei Ablenkspiegel 9 und 9', die um in Fig.1 nicht gezeigte Achsen kippbar gelagert sind. Zur einfacheren Darstellung sind in Fig.1 die Spiegel 9 und 9' parallel ausgerichtet, tatsächlich jedoch verlaufen die Kippachsen orthogonal zueinander und zur optischen Achse der Eingangsoptik 6, so dass durch Kippen des ersten Spiegels 9 das Strahlenbündel 3 in Y-Richtung und durch Kippen des zweiten Spiegels 9' in der dazu orthogonalen X-Richtung abgelenkt wird. Die Spiegel 9 und 9' werden durch Aktoren 10 bzw. 10' angetrieben, die über Signalwege (durch Pfeile angedeutet) mit einer Steuereinrichtung verbunden sind. Die Steuereinrichtung gibt entsprechend der gewünschten Fokuslage in lateraler Richtung Steuersignale an die Aktoren 10 und 10' ab, die daraufhin das Kippen der Spiegel 9 und 9'veranlassen.

Die Eingangsoptik 6 weist eine relativ zu der Ablenkeinrichtung 7 bewegbare Linse 11 negativer Brechkraft auf sowie eine Sammellinse 12. Die Linse 11 ist mit einer Geradführung 13 verbunden, durch die sie mit veränderlichem optisch wirksamem Abstand zur Ablenkeinrichtung 7 verschiebbar ist. Zur Auslösung der Verschiebung der Linse 11 parallel zu ihrer optischen Achse dient zum Beispiel ein Linearantrieb 14, der ebenfalls mit der nicht gezeigten Steuereinrichtung verbunden ist. Diese generiert in Abhängigkeit von der gewünschten Fokuslage in Z-Richtung Steuersignale an den Linearantrieb 14.

Aufgrund der konstruktiven Ausbildung der Linse 11 und der Sammellinse 12 wirkt die Eingangsoptik 6 als Expander, der den Durchmesser des Strahlenbündels 3 aufweitet. Tritt also ein paralleles Strahlenbündel 3 mit einem Durchmesser d1 in die Eingangsoptik 6 ein, tritt aus der Eingangsoptik 6 ein paralleles Strahlenbündel 3 mit einem Durchmesser d2>d1 aus.

Das Objektiv 8 ist als eine feststehende Linse 15 dargestellt, es fokussiert das aus der Eingangsoptik 6 ausgetretene Strahlenbündel 3 in eine mittels der Linse 11 und der Ablenkeinrichtung 7 vorgegebene Position im Bereich der Augenhornhaut.

Die Lage des Fokus F in der Tiefe des Bereichs der Augenhornhaut wird durch das Verschieben der Linse 11 entlang ihrer optischen Achse bestimmt. Die seitliche Position des Fokus F wird mit der Ablenkeinrichtung 7 eingestellt.

Fig.2 zeigt eine Variante der Anordnung nach Fig.1, wobei hier im Strahlengang zwischen der Ablenkeinrichtung 7 und dem Objektiv 8 ein Strahlteiler 16 angeordnet ist, mit dem das von der Augenhornhaut ausgehende und mit dem Objektiv 8 zu einem Beobachtungsstrahlenbündel 17 geformte Licht teilweise ausgekoppelt und in Richtung auf einen nicht gezeigten Tubus mit Okular oder eine ebenfalls nicht gezeigte Kamera abgelenkt wird, so dass eine Mitbeobachtung der Augenhornhaut während der Untersuchung oder Behandlung möglich ist.

Weitere Details zu diesem Stand der Technik sind der Veröffentlichung DE 10 2005 013 949 A1 zu entnehmen.

Die der Erfindung zugrunde liegende Aufgabe wird abweichend vom Stand der Technik gelöst, indem das Objektiv 8 aus mehreren Linsengruppen besteht und so ausgebildet ist, dass es selbst oder mindestens eine Linsengruppe relativ zum Auge veränderbar sind, und zwar so, dass diese Abstandsänderung das Verschieben der Fokusposition aus dem Bereich der Augenhornhaut in den Bereich der Augenlinse und umgekehrt bewirkt.

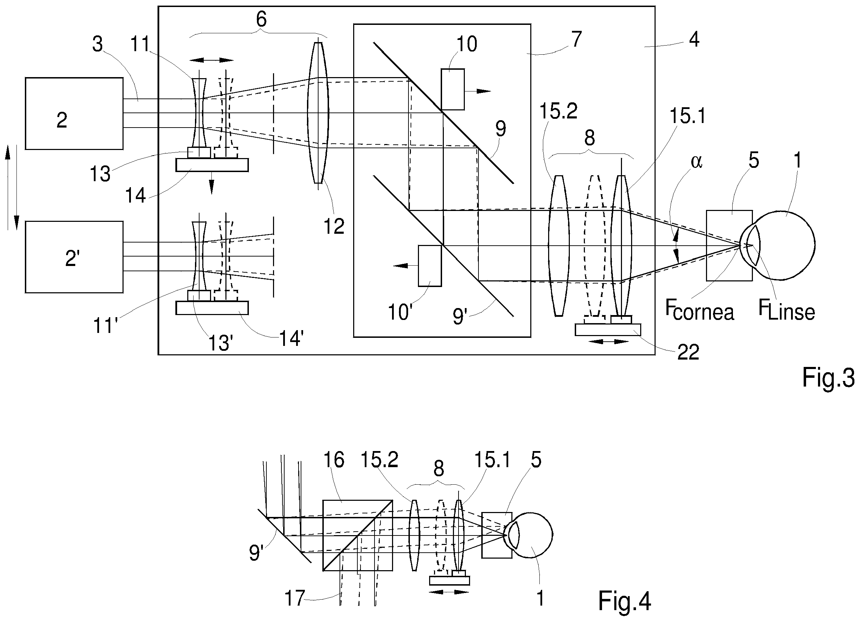

Fig.3 zeigt eine Darstellung des erfindungsgemäßen Gerätes in einer ersten bevorzugten Ausführungsform. Hier besteht das Objektiv 8 aus zwei Linsengruppen 15.1 und 15.2, die der Übersichtlichkeit halber symbolisch als Linsen dargestellt sind. Die Linsengruppe 15.2 befindet sich an einer festen Position im Strahlengang, wogegen die Linsengruppe 15.1 in Richtung der optischen Achse verschiebbar angeordnet und zu diesem Zweck mit einer Geradführung gekoppelt ist, die ihrerseits beispielsweise mit einem Linearantrieb 22 verbunden ist, der die Verschiebebewegung auslöst und diesbezüglich von einer (nicht dargestellten) Steuereinrichtung angesteuert wird.

Die Weite der Verschiebung der Linsengruppe 15.1 ist zwar grundsätzlich vom Ansteuersignal abhängig, sie ist jedoch vorteilhaft durch zwei Endlagen definiert, von denen in Fig.3 eine erste Endlage durch die in Vollstrich gezeichnete und die zweite Endlage durch die mit unterbrochenem Strich gezeichnete Linsengruppe 15.1 kenntlich gemacht ist.

Diese mit den Endlagen fest vorgegebene Verschiebeweite entspricht dem Versetzen der Fokusposition aus dem Bereich der Augenhornhaut in den Bereich der Augenlinse und umgekehrt, womit erreicht wird, dass die erfindungsgemäße Anordnung sowohl für Untersuchungen und Behandlungen der Augenhornhaut als auch der Augenlinse genutzt werden kann. Da Patientenaugen naturgemäß nicht alle gleich groß sind, ist in einer Ausführungsvariante der Erfindung vorgesehen, dass die Verschiebeweite individuell vom Patienten abhängt. In diesem Fall gibt es nur einen festen Anschlag für das Arbeiten in der Augenhornhaut und eine kontinuierliche Einstellgrenze für die Augenlinse.

Befindet sich die Linsengruppe 15.1 in der ersten Endlage, kann die Fokusposition mittels der Linse 11 in Z-Richtung und mittels der Ablenkeinrichtung 7 in X- und Y-Richtung so verändert werden, dass damit zwar die Antastung aller gewünschte Ziele innerhalb des Bereichs der Augenlinse möglich ist, jedoch nicht darüber hinaus. Dies trifft sinngemäß für die zweite Endlage zu und damit für die Möglichkeit der Antastung aller gewünschten Ziele innerhalb des Bereiches der Augenhornhaut.

Die Verschiebung der Linsengruppe 15.1 zwischen den beiden Endlagen bzw. die Befehlsauslösung für diese Verschiebung entspricht somit der Umschaltung des erfindungsgemäßen Gerätes zwischen zwei Betriebsmodi, wobei in dem einen Betriebsmodus das Gerät zur Untersuchung und Behandlung der Augenlinse, in dem zur Untersuchung und Behandlung der Augenhornhaut nutzbar ist.

Es ist vorgesehen, den Bündeldurchmesser 3' zu variieren, um damit den augenseitigen Öffnungswinkel α dem jeweiligen Betriebsmodus anzupassen. Wird nämlich das Verhältnis der Beträge der Brechkräfte der Linse 11 und der Sammellinse 12 verringert, wird auch der Durchmesser des aus der Sammellinse 12 austretenden parallelen Strahlenbündels 3' kleiner und in Folge dessen auch der augenseitige Öffnungswinkel a. Ein kleinerer Öffnungswinkel α ist für die Fokuspositionierung an oder in der Augenlinse von Vorteil, da der Augenlinse die Pupille als einschränkende Apertur für das in die Augenlinse eintretende konvergierende Strahlenbündel 3 vorgelagert ist und die optische Korrektion verbessert wird.

Die Änderung des Verhältnis der Brechkräfte in der Gruppe 6, und somit des Durchmessers des Bündels 3' erfolgt - wie in Fig.3 dargestellt - durch Austausch mehrerer, hier beispielsweise zweier Linsen 11 und 11' gegeneinander, die unterschiedliche negative Brechkraft haben. Dabei werden entweder nur die Linsen 11 und 11' gegeneinander ausgetauscht oder die aus Strahlungsquelle 2, Linse 11, Linsenhalter 13 und Linearantrieb 14 bestehende Baugruppe gegen eine aus Strahlungsquelle 2', Linse 11', Linsenhalter 13' und Linearantrieb 14' bestehende Baugruppe ausgetauscht.

Optional können die Linse 11 und die Sammellinse 12 auch gemeinsam als Linsenpaar ausgetauscht werden, um denselben Effekt zu erzielen.

Die Änderung des Divergenzwinkels kann in einer ersten Variante mit dem Umschalten zwischen den Betriebsmodi zwangsgekoppelt sein und damit automatisch erfolgen, oder in einer alternativen zweiten Variante in Abhängigkeit von der Größe des zu untersuchenden bzw. zu behandelnden Auges vorgenommen werden. Als Maß für die Augengröße kann beispielsweise die Vorderkammertiefe gelten, die vor Beginn einer Untersuchung separat ermittelt wird. In erster Linie wird aus der Vermessung des Auges die Verschiebung der Linse 15.1 abgeleitet. Die Anpassung des Bündeldurchmessers des Bündels 3' durch das Brechzahlverhältnis innerhalb der Optik 6 kann automatisch im Zusammenhang mit dem Umschalten der Betriebsmodi vorgesehen sein.

Fig.4 zeigt eine Variante der Anordnung nach Fig.3 mit einem zwischen der Ablenkeinrichtung 7 und dem Objektiv 8 angeordneten Strahlteiler 16, der dazu dient, Licht des jeweils vom Auge kommenden Beobachtungsstrahlenbündels 17 auszukoppeln und in Richtung auf einen nicht gezeigten Tubus mit Okular oder eine ebenfalls nicht gezeigte Kamera zu lenken, um eine Mitbeobachtung der untersuchten oder behandelten Stelle im Auge zu ermöglichen.

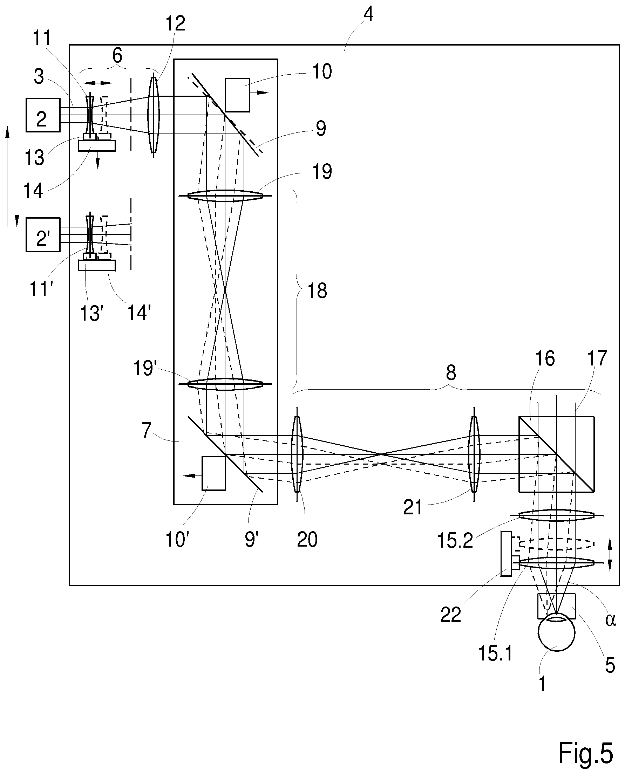

Fig.5 zeigt eine weitere bevorzugte Ausführungsform der Erfindung. In Fig.5 wurden für gleiche Komponenten wie in den vorhergehenden Ausführungsbeispielen auch wieder die gleichen Bezugszeichen verwendet.

Hier ist in den Strahlengang zwischen den Spiegeln 9 und 9' eine Pupillenoptik 18 eingeordnet. Diese weist zwei Sammellinsen 19 und 19' auf, welche das von dem Spiegel 9 abgelenkte Strahlenbündel 3 auf den Spiegel 9' abbilden, wobei zwischen den Sammellinsen 19 und 19' ein reelles Zwischenbild in Luft entsteht. Auf diese Weise ergibt sich eine feste Lage der Pupille, die eine optisch günstige Auslegung des Objektivs 8 ermöglicht. Darüber hinaus kann aufgrund der Abbildung der Spiegel 9 und 9' ineinander die Größe des Spiegels 9' klein gehalten werden. Die Spiegel 9 und 9' haben beispielsweise die Form von Ellipsen.

Das Objektiv 8 ist in dieser Ausführungsform der Erfindung insofern mehrteilig aufgebaut, als es über eine Eintrittslinsengruppe 20, eine Tubuslinse 21, und die Linsengruppen 15.1 und 15.2 verfügt.

Das von der Ablenkeinrichtung 7 lateral abgelenkte Strahlenbündel 3 tritt in die Eintrittslinsengruppe 20 ein und wird von dieser in ein reelles Zwischenbild fokussiert. Die Tubuslinse 21 bildet das Zwischenbild nach Unendlich ab. Ein Strahlteiler 16 lenkt das Strahlenbündel 3 zu den Linsengruppen 15.1 und 15.2 hin ab.

Die Verstellung der Fokuslage in den Koordinatenrichtungen X, Y und Z erfolgt ebenso wie im vorhergehenden Ausführungsbeispiel beschrieben. Auch das Umschalten zwischen zwei Betriebmodi durch das Verschieben der Linsengruppe 15.1 zwischen zwei Endlagen erfolgt wie oben beschrieben, wobei in dem einen Betriebsmodus das Gerät zur Untersuchung und Behandlung der Augenlinse, in dem anderen zur Untersuchung und Behandlung der Augenhornhaut konfiguriert ist.

Vorteilhaft sind alle optischen Komponenten so ausgelegt, dass die reellen Zwischenbilder in Luft liegen und daher sehr intensive Laserstrahlung nicht zu optischen Durchbrüchen in optischen Komponenten führen kann.

Die Strahlungsquelle 2, die Eingangsoptik 6 und das Objektiv 8 sind in allen Ausführungsformen der Erfindung so ausgelegt, dass das Strahlungsbündel 3 im Fokus einen Durchmesser kleiner als 5 Mikrometer hat. Das Objektiv 8 hat vorzugsweise eine Apertur größer als 0,35.

### Bezugszeichenliste

- 1: Auge
- 2: Strahlungsquelle
- 3: Strahlungsbündel
- 4: Scanvorrichtung
- 5: Kontaktglas
- 6: Eingangsoptik
- 7: Ablenkeinrichtung
- 8: Objektiv
- 9,9': Spiegel
- 10, 10': Aktoren
- 11: erste Linse
- 12: Sammellinse
- 13: Linsenhalter
- 14: Linearantrieb
- 15.1, 15.2: Linsengruppen
- 16: Strahlteiler
- 17: Beobachtungsstrahlenbündel
- 18: Pupillenoptik
- 19,19': Sammellinsen
- 20: Eintrittslinsengruppe
- 21: Tubuslinse
- 22: Linearantrieb

## Patentansprüche

1. Ophthalmologisches Gerät zur Anwendung von Laserstrahlung im Auge zur Untersuchung und/oder laserchirurgischen Behandlung der Augenhornhaut und der Augenlinse, umfassend:
- einen Femtosekundenlaser als Strahlungsquelle (2) für die Laserstrahlung,
- eine Eingangsoptik (6) mit einer Linse (11) und einer Sammellinse (12),
- ein Objektiv (8), aus dem ein Strahlenbündel (3) fokussiert in Richtung auf das Auge austritt,
- optische Baugruppen,
- die dem Objektiv (8) in Strahlungsrichtung gesehen vorgeordnet sind und
- die in Wirkverbindung mit dem Objektiv zur Variation der Fokusposition in der Koordinatenrichtung X,Y und Z wahlweise innerhalb des Bereiches der Augenhornhaut oder innerhalb des Bereiches der Augenlinse vorgesehen sind,
- wobei das Objektiv (8) selbst relativ zum Auge beweglich ist oder mindestens eine Linsengruppe (15.1, 15.2) aufweist, deren Lage relativ zum Auge veränderbar ist,
- **dadurch gekennzeichnet, dass**
mit der Variation der Lage der mindestens einen Linsengruppe (15.1, 15.2) oder des gesamten Objektivs (8) die Fokusposition aus dem Bereich der Augenhornhaut in den Bereich der Augenlinse und umgekehrt versetzbar ist,
- wobei der Durchmesser des aus der Eingangsoptik (6) austretenden Strahlenbündels (3) durch Variation des Verhältnis der Beträge der Brechkräfte der Linse (11) und der Sammellinse (12) variierbar und somit der augenseitige Öffnungswinkel α veränderbar ist,
- und wobei der augenseitige Öffnungswinkel α bei einer Fokussierung auf den Bereich der Augenlinse kleiner ist als bei einer Fokussierung auf den Bereich der Augenhornhaut.

2. Ophthalmologisches Gerät nach Anspruch 1, bei welchem
- die Linse (11) dem Objektiv (8) in Strahlungsrichtung verschiebbar, und mit negativer Brechkraft vorgeordnet ist, mit deren Verschiebung die Fokusposition in der Koordinatenrichtung Z variierbar ist, und
- dem Objektiv (8) eine Ablenkeinrichtung (7) zur Variation der Fokusposition in den Koordinatenrichtungen X,Y vorgeordnet ist, wobei
- die Ablenkeinrichtung (7) zwischen der Linse (11) und dem Objektiv (8) angeordnet ist.

3. Ophthalmologisches Gerät nach Anspruch 1 oder 2, bei dem die Veränderung des augenseitigen Öffnungswinkels α mit dem Versetzen der Fokusposition aus dem Bereich der Augenhornhaut in den Bereich der Augenlinse und umgekehrt verbunden ist.

4. Ophthalmologisches Gerät nach Anspruch 3, bei dem der Betrag des Fokusversatzes zwischen Augenhornhaut und Augenlinse in Abhängigkeit von der individuellen Vorderkammertiefe vorgegeben ist.

5. Ophthalmologisches Gerät nach einem der vorgenannten Ansprüche, bei dem ein Kontaktglas (5) mit einer konkaven Kontaktfläche auf die Augenhornhaut aufgesetzt ist, um eine Augenbewegung zu unterdrücken.

6. Ophtalmologisches Gerät nach einem der Ansprüche 2 bis 5, bei welchem der Linse (11) mit negativer Brechkraft in Strahlrichtung die Sammellinse (12) nachgeordnet ist.

7. Ophthalmologisches Gerät nach einem der Ansprüche 2 bis 6, bei dem die Linse (11) mit einem Linearantrieb (22) gekoppelt ist.

8. Ophthalmologisches Gerät nach einem der vorhergehenden Ansprüche, bei dem zur Variation des Brechkraftverhältnisses innerhalb der Eingangsoptik (6) mehrere Linsen (11, 11') oder Linsenpaare (11, 11', 12, 12') gegeneinander austauschbar sind.

9. Ophthalmologisches Gerät nach einem der vorhergehenden Ansprüche, bei dem das Objektiv (8) zwei Linsengruppen (15.1, 15.2) aufweist, deren Lage relativ zum Auge veränderbar ist, und außer diesen eine Eintrittslinsengruppe (20) zur Erzeugung eines reellen Zwischenbildes der Strahlungsquelle (2) aufweist.

10. Ophthalmologisches Gerät nach einem der vorhergehenden Ansprüche, bei dem die Ablenkeinrichtung (7) zwei voneinander beabstandete und relativ zueinander kippbare Spiegel (9, 9') aufweist, wobei vorzugsweise zwischen den Spiegeln (9, 9') eine zwei Sammellinsen (19, 19') umfassende Pupillenoptik angeordnet ist.

11. Ophthalmologisches Gerät nach einem der vorhergehenden Ansprüche, bei dem im Strahlengang zwischen der Ablenkeinrichtung (7) und dem Objektiv (8) ein Strahlteiler (16) angeordnet ist zwecks Auskopplung eines auf ein Okular oder eine Kamera gerichteten Teilstrahlengangs.

12. Ophthalmologisches Gerät nach einem der vorhergehenden Ansprüche, bei dem die reellen Zwischenbilder der Strahlungsquelle (2) in Luft liegen.

13. Ophthalmologisches Gerät nach einem der vorhergehenden Ansprüche, bei dem
- die Variationsbreite der Fokuslage in der Koordinatenrichtung Z im Bereich der Augenhornhaut größer als 0,5 mm ist,
- die Variationsbreite der Fokuslage in der Koordinatenrichtung Z im Bereich der Augenlinse größer als 2 mm ist,
- der typische Felddurchmesser der Augenhornhaut bis 11 mm beträgt,
- der typische Felddurchmesser der Augenlinse bis 7 mm beträgt,
- der typische Fokusdurchmesser der Augenhornhaut bis < 3µm beträgt, und
- der typische Fokusdurchmesser der Augenlinse bis < 5 µm beträgt

## Claims

1. Ophthalmological apparatus for applying laser radiation into the eye for the examination and/or laser-surgical treatment of the cornea of the eye and the crystalline lens, comprising:
- a femtosecond laser as a radiation source (2) for the laser radiation,
- an input optical unit (6) having a lens (11) and a converging lens (12),
- an objective (8), from which a beam (3) emerges in focus in the direction of the eye,
- optical assemblies,
- which are disposed upstream of the objective (8) in the radiation direction and
- which are provided functionally connected to the objective for the purposes of varying the focal position in the coordinate directions X, Y and Z, selectively within the region of the cornea of the eye or within the region of the crystalline lens,
- the objective (8) itself being movable relative to the eye or comprising at least one lens group (15.1, 15.2), the relative position of which is changeable in relation to the eye,
- **characterized in that**
- the focal position can be displaced from the region of the cornea of the eye into the region of the crystalline lens, and vice versa, by varying the relative position of the at least one lens group (15.1, 15.2) or of the entire objective (8),
- with the diameter of the beam (3) emerging from the input optical unit (6) being variable by varying the ratio of the absolute values of the refractive powers of the lens (11) and the converging lens (12) and consequently the eye-side aperture angle α being changeable,
- and with the eye-side aperture angle α being smaller when focussing on the region of the crystalline lens than when focussing on the region of the cornea of the eye.

2. Ophthalmological apparatus according to Claim 1, wherein
- the lens (11) is disposed upstream of the objective (8) in the beam direction in displaceable fashion and with negative refractive power, the displacement of said lens rendering the focal position variable in the coordinate direction Z, and
- a deflection device (7) for varying the focal position in the coordinate directions X, Y is disposed upstream of the objective (8), with
- the deflection device (7) being arranged between the lens (11) and the objective (8) .

3. Ophthalmological apparatus according to Claim 1 or 2, wherein the change in the eye-side aperture angle α is connected to the displacement of the focal position from the region of the cornea of the eye into the region of the crystalline lens, and vice versa.

4. Ophthalmological apparatus according to Claim 3, wherein the absolute value of the focal offset between cornea of the eye and crystalline lens is specified on the basis of the individual anterior chamber depth.

5. Ophthalmological apparatus according to any one of the preceding claims, wherein a contact glass (5) with a concave contact surface is placed onto the cornea of the eye in order to suppress an eye movement.

6. Ophthalmological apparatus according to any one of Claims 2 to 5, wherein the converging lens (12) is disposed downstream in the beam direction of the lens (11) with negative refractive power.

7. Ophthalmological apparatus according to any one of Claims 2 to 6, wherein the lens (11) is coupled to a linear drive (22).

8. Ophthalmological apparatus according to any one of the preceding claims, wherein a plurality of lenses (11, 11') or lens pairs (11, 11', 12, 12') are exchangeable against one another for the purposes of varying the refractive power ratio within the input optical unit (6).

9. Ophthalmological apparatus according to any one of the preceding claims, wherein the objective (8) comprises two lens groups (15.1, 15.2), the relative position of which being changeable in relation to the eye, and, in addition to these, comprises an entrance lens group (20) for producing a real intermediate image of the radiation source (2).

10. Ophthalmological apparatus according to any one of the preceding claims, wherein the deflection device (7) comprises two spaced apart mirrors (9, 9') that are tiltable relative to one another, with a pupil optical unit comprising two converging lenses (19, 19') preferably being arranged between the mirrors (9, 9').

11. Ophthalmological apparatus according to any one of the preceding claims, wherein a beam splitter (16) is arranged in the beam path between the deflection device (7) and the objective (8) for the purposes of output coupling a partial beam path that is directed at an eyepiece or a camera.

12. Ophthalmological apparatus according to any one of the preceding claims, wherein the real intermediate images of the radiation source (2) are located in air.

13. Ophthalmological device according to any one of the preceding claims, wherein
- the range of variation of the relative focal position in the coordinate direction Z is greater than 0.5 mm in the region of the cornea of the eye,
- the range of variation of the relative focal position in the coordinate direction Z is greater than 2 mm in the region of the crystalline lens,
- the typical field diameter in the cornea of the eye is up to 11 mm,
- the typical field diameter in the crystalline lens is up to 7 mm,
- the typical focal diameter in the cornea of the eye is up to < 3 µm, and
- the typical focal diameter in the crystalline lens is up to < 5 µm.

## Revendications

1. Appareil ophtalmologique pour l'application d'un rayonnement laser dans l'œil pour un examen et/ou un traitement par chirurgie laser de la cornée et du cristallin, comprenant :
- un laser femtoseconde en tant que source de rayonnement (2) pour le rayonnement laser,
- une optique d'entrée (6) comportant une lentille (11) et une lentille convergente (12),
- un objectif (8) duquel sort un faisceau de rayonnement (3) focalisé dans la direction de l'œil,
- des blocs optiques,
- qui sont disposés en amont de l'objectif (8), vu dans la direction du rayonnement et
- qui sont disposés en liaison active avec l'objectif pour faire varier la position focale dans la direction des coordonnées X, Y et Z, au choix à l'intérieur de la région de la cornée ou à l'intérieur de la région du cristallin,
- dans lequel l'objectif (8) lui-même est mobile par rapport à l'œil ou comporte au moins un groupe de lentilles (15.1, 15.2) dont la position par rapport à l'œil peut être modifiée,
- **caractérisé en ce que**
- la variation de la position dudit au moins un groupe de lentilles (15.1, 15.2) ou de la totalité de l'objectif (8) permet de déplacer la position focale de la région de la cornée vers la région du cristallin et inversement,
- dans lequel le diamètre du faisceau de rayons (3) sortant de l'optique d'entrée (6) peut être modifié en faisant varier le rapport des valeurs des puissances de réfraction de la lentille (11) et de la lentille convergente (12) et ainsi de modifier l'angle d'ouverture α du côté de l'œil,
- et dans lequel l'angle d'ouverture α du côté de l'œil est plus petit lors d'une focalisation sur la région du cristallin que lors d'une focalisation sur la région de la cornée.

2. Appareil ophtalmologique selon la revendication 1, dans lequel
- la lentille (11) est disposée en amont de l'objectif (8) de manière à ce qu'elle puisse être déplacée dans la direction du rayonnement et avec une puissance de réfraction négative, son déplacement permettant de faire varier la position focale dans la direction de coordonnées Z, et
- un dispositif de déviation (7) est disposé en amont de l'objectif (8) pour faire varier la position focale dans les directions de coordonnées X, Y, dans lequel
- le dispositif de déviation (7) est disposé entre la lentille (11) et l'objectif (8).

3. Appareil ophtalmologique selon la revendication 1 ou 2, dans lequel la modification de l'angle d'ouverture α du côté de l'œil est liée au déplacement de la position focale depuis la région de la cornée vers la région du cristallin et inversement.

4. Appareil ophtalmologique selon la revendication 3, dans lequel la valeur du décalage du foyer entre la cornée et le cristallin est prédéfinie en fonction de la profondeur individuelle de la chambre antérieure.

5. Appareil ophtalmologique selon l'une des revendications précédentes, dans lequel un verre de contact (5) présentant une surface de contact concave est placé sur la cornée afin de supprimer un mouvement oculaire.

6. Appareil ophtalmologique selon l'une des revendications 2 à 5, dans lequel la lentille convergente (12) est disposée en aval de la lentille (11) de puissance négative dans la direction du faisceau.

7. Appareil ophtalmologique selon l'une des revendications 2 à 6, dans lequel la lentille (11) est couplée à un dispositif d'entraînement linéaire (22).

8. Appareil ophtalmologique selon l'une des revendications précédentes, dans lequel plusieurs lentilles (11, 11') ou paires de lentilles (11, 11', 12, 12') sont interchangeables pour faire varier le rapport de puissance de réfraction à l'intérieur de l'optique d'entrée (6).

9. Appareil ophtalmologique selon l'une des revendications précédentes, dans lequel l'objectif (8) comporte deux groupes de lentilles (15.1, 15.2) dont la position par rapport à l'œil est modifiable, et comporte en plus de ceux-ci un groupe de lentilles d'entrée (20) destiné à produire une image intermédiaire réelle de la source de rayonnement (2).

10. Appareil ophtalmologique selon l'une des revendications précédentes, dans lequel le dispositif de déviation (7) comporte deux miroirs (9, 9') espacés l'un de l'autre et pouvant basculer l'un par rapport à l'autre, dans lequel une optique pupillaire comprenant deux lentilles convergentes (19, 19') est de préférence disposée entre les miroirs (9, 9').

11. Appareil ophtalmologique selon l'une des revendications précédentes, dans lequel un séparateur de faisceau (16) est disposé sur le chemin de faisceau entre le dispositif de déviation (7) et l'objectif (8) en vue du découplage d'un chemin de faisceau partiel dirigé vers un oculaire ou une caméra.

12. Appareil ophtalmologique selon l'une des revendications précédentes, dans lequel les images intermédiaires réelles de la source de rayonnement (2) se situent dans l'air.

13. Appareil ophtalmologique selon l'une des revendications précédentes, dans lequel
- l'amplitude de variation de la position focale dans la direction de coordonnées Z dans la région de la cornée est supérieure à 0,5 mm,
- l'amplitude de variation de la position focale dans la direction de coordonnées Z dans la région du cristallin est supérieure à 2 mm,
- le diamètre de champ typique de la cornée est d'au plus 11 mm,
- le diamètre de champ typique de la lentille oculaire est d'au plus 7 mm,
- le diamètre de foyer typique de la cornée est d'au plus < 3µm, et
- le diamètre de foyer typique de la lentille oculaire est d'au plus < 5µm.
